# EUROPEAN PATENT SPECIFICATION

(11) **EP 2 624 915 B1**
(45) Date of publication and mention of the grant of the patent: **21.11.2018**
(21) Application number: 11830150.6
(22) Date of filing: 05.10.2011
(51) Int. Cl.: A61N 5/10, A61B 5/055

(54) **IMAGE GUIDED RADIATION THERAPY SYSTEM AND SHIELDED RADIOFREQUENCY DETECTOR COIL FOR USE THEREIN**
BILDGESTEUERTES STRAHLUNGSTHERAPIESYSTEM UND ABGESCHIRMTE RADIOFREQUENZ-DETEKTORSPULE ZUR VERWENDUNG DARIN
SYSTÈME DE RADIOTHÉRAPIE GUIDÉ PAR L'IMAGE ET BOBINE DE DÉTECTION DE RADIOFRÉQUENCE BLINDÉE DESTINÉE À ÊTRE UTILISÉE AVEC CELUI-CI

(30) Priority: 24.05.2011 US 201161489550 P; 05.10.2010 US 390172 P
(43) Date of publication of application: 14.08.2013
(73) Proprietor: Alberta Health Services, Edmonton, AB T5J 3H1 (CA)
(72) Inventor: Rathee, Satyapal, Edmonton, AB T6R 2Y1 (CA); Burke, Benjamin, Edmonton, AB T6G 0T9 (CA); Ghila, Andrei, Edmonton AB T6K 1A7 (CA); Fallone, Gino, Edmonton, AB T6R 2G6 (CA)
(74) Representative: Naismith, Robert Stewart
(86) International application number: PCT/CA2011/001116
(87) International publication number: WO 2012/045153

(56) References cited:
- US-A1- 2007 152 668
- US-A1- 2009 021 255
- US-B1- 6 701 176
- US-B1- 6 701 176
- US-B1- 7 427 265
- BURKE B ET AL: "Radiation induced currents in MRI RF coils: application to linac/MRI integration; Radiation induced currents in MRI RF coils", PHYSICS IN MEDICINE AND BIOLOGY, INSTITUTE OF PHYSICS PUBLISHING, BRISTOL GB, vol. 55, no. 3, 7 February 2010 (2010-02-07), pages 735-746, XP020171759, ISSN: 0031-9155

## Description

### Field of the Invention

The present application relates generally to radiation therapy and in particular to an image guided radiation therapy system and shielded MRI radiofrequency detector coil for use therein.

### Background of the Invention

Image guidance for radiation therapy is an active area of investigation and technology development. Current radiotherapy practice utilizes highly conformal radiation portals that are directed at a precisely defined target region. This target region consists of the Gross Tumour Volume (GTV), the Clinical Target Volume (CTV) and the Planning Target Volume (PTV). The GTV and CTV consist of gross tumour disease and the subclinical microscopic extension of the gross disease. During radiation treatments, these volumes must be irradiated at a sufficient dose in order to give an appropriate treatment to the patient. Because of the uncertainty in identifying this volume at the time of treatment, and due to unavoidable patient and tumour motion, an enlarged PTV is typically irradiated.

Because a volume that is larger than the biological extent of the disease and therefore healthy tissue is typically irradiated, there is an increased risk of complications. Thus, it is desirable to conform the radiation beam to the GTV and CTV only, and to provide an imaging method to assist in the placement of the radiation beam on this volume at the time of treatment. This technique is known as Image Guided Radiation Therapy (IGRT).

Commercially available techniques that are available for IGRT typically use x-ray or ultrasound imaging technology to produce planar x-ray, computed tomography, or 3D ultrasound images. Furthermore, fiducial markers can be used in conjunction with these imaging techniques to improve contrast. However, fiducial markers must be placed using an invasive technique, and are thus less desirable. IGRT techniques based on x-rays or ultrasound are not ideally suited to IGRT. For example, x-rays suffer from low soft tissue contrast and are not ideally suited to imaging tumours. Furthermore, x-ray based techniques use ionizing radiation and result in a supplemental dose deposit to the patient. Ultrasound cannot be utilized in all locations of the body. Finally, both x-ray and ultrasound based IGRT techniques are difficult to integrate into a linear accelerator such that they can provide images in any imaging plane in real time at the same moment as the treatment occurs.

In order to overcome these difficulties, it has been proposed to integrate a radiotherapy system with a Magnetic Resonance Imaging (MRI) device. For example, PCT Patent Application Publication No. WO 2007/045076 to Fallone et al., assigned to the assignee of the present application, describes a medical linear accelerator that is combined with a bi-planar permanent magnet suitable for MRI. As is well known, MRI offers excellent imaging of soft tissues, and can image in any plane in real time.

An MRI functions by providing a strong and homogeneous magnetic field that aligns the nuclear magnetic moments of target nuclei. For example, hydrogen nuclei (protons) are the most common imaging target in MRI. In the presence of the magnetic field, the magnetic moments of the nuclei align with the homogeneous magnetic field and oscillate at a frequency determined by the field strength, known as the Larmor frequency. This alignment can be perturbed using a radiofrequency (RF) pulse, such that the magnetization flips from the direction of the magnetic field (B₀ field) to a perpendicular direction, and thus exhibits transverse magnetization. When the nuclei reverts back to its original state, the transverse magnetic moment decays to zero, while the longitudinal magnetic moment increases to its original value. Different soft tissues exhibit different transverse and longitudinal relaxation times. A specific magnetic field strength is applied to a small sample of tissue utilizing gradient magnetic coils, and images of these soft tissues can be formed by first generating a specific sequence of perturbing RF pulses and then analyzing the signals that are emitted by the nuclei as they return to their original magnetization state after being perturbed by the pulses.

A medical linear accelerator functions by using a cylindrical waveguide that is excited in a TM₀₁₀ mode such that the electric field lies upon the central axis of the waveguide. The phase velocity of the structure is controlled by introducing septa into the waveguide which form cavities. The septa have small holes at their centre to allow passage of an electron beam. Septa have the further advantage that they intensify the electric field at the center of the waveguide such that field gradients in the MeV/m range are available for RF input power that is in the MW range. Electrons are introduced into one end of the accelerating structure, and are then accelerated to MeV energies by the central electric field of the accelerating waveguide. These electrons are aimed at a high atomic number target, and the electronic energy is converted in high energy x-rays by the bremsstrahlung process. The waveguide is typically mounted on a C-arm gantry such that the central axis of the waveguide is parallel to the ground. This waveguide rotates around a patient, which lies at the central axis of rotation. The medical accelerator utilizes a system employing a 270° bending magnet such that the radiation beam generated by the waveguide is focused at a point on the central axis of rotation known as the isocentre.

There are several significant technological challenges associated with the integration of a linear accelerator with an MRI device. U.S. Patent No. 6,366,798 to Green, PCT Patent Application Publication No. WO 2004/024235 to Lagendijk,, U.S. Patent No. 6,862,469 to Bucholz et al., PCT Patent Application Publication No. WO 2006/136865 to Kruip et al., U.S. Patent Application Publication No. 2005/0197564 to Dempsey, PCT Patent Application Publication No. WO 2009/155700 to Fallone et al., U.S. Patent Application Publication No. 2009/0149735 to Fallone et al., U.S. Patent Application Publication No. 2009/0147916 to Fallone et al., and PCT Patent Application Publication No. 2009/155691 to Fallone et al. disclose various systems and techniques that address some of the challenges.

However, while the documents referred to above provide various advancements, there are technological challenges that are yet to be satisfactorily addressed.

Some challenges are due to the pulsed power nature of the linear accelerator. In order to supply sufficient RF power (on the order of Mega-Watts MWs) to the accelerating waveguide to produce an effective treatment beam, medical linear accelerators operate in a pulsed power mode where high voltage is converted to pulsed power using a pulse forming network (PFN). The process of generating high voltage pulses involves sudden starting and stopping of large currents in the modulation process, and in addition to producing a pulsed treatment beam can in turn give rise to radiofrequency emissions whose spectrum can overlap the Larmor frequency of the hydrogen nuclei within the imaging subject. The overlapping radiofrequency emissions of the pulse forming network can interfere with the signals emitted by these nuclei as they relax, thus deteriorating the image forming process of the MRI.

Additional problems are due to the pulsed treatment beam being often incident on the MRI radiofrequency detector coil or coils used to detect the radiofrequency signals generated while nuclei are relaxing. This causes radiation induced effects, classed generally as follows: (a) instantaneous-coincides with linac radiation pulses and includes a radiation induced current (RIC) in the detector coil, (b) accumulative-occurs over time and could include damage to the RF detector coil and associated hardware and (c) dosimetric-modification of the patient skin dose caused by the presence of the RF detector coil in the magnetic field.

Where instantaneous radiation induced effects are concerned, it is possible to synchronize the acquisition process so that the radiation pulse does not occur at the exact same time as imaging. However, such a restriction can limit the adaptability of the system. As such, RIC in the detector coil or coils can interfere with the fidelity of imaging signals in the detector coil or coils. This problem manifests itself because, when irradiated with high-energy (megavoltage) photons, the high-energy electrons produced in Compton interactions are likely to escape the thin coil material, such as copper strips known to be used in MRI RF coils. If there is no influx of electrons to balance this effect, a net positive charge is created in the material. Therefore, if the coil material is part of an electrical circuit, a current induced by the radiation will begin to flow in order to neutralize this charge imbalance. Meyer et al (1956) reported in 1956 on the RIC seen in polyethylene and Teflon upon exposure to x-rays from a 2 MeV Van de Graaff generator and a 60Co beam. Johns et al (1958) reported the RIC due to the 60Co beam in parallel plate ionization chambers providing RIC as the basis of the polarity effect observed in these chambers. Several authors have published reports on RIC in varying materials when exposed to pulsed radiation (Degenhart and Schlosser 1961, Sato et al 2004, Abdel-Rahman et al 2006), which are of particular relevance to this work.

The document US 2007/152668 A1 discloses such an MRI RF coil covered on one side by an electrically grounded dielectric material.

Since the premise of linac-MRI integration for image guided radiotherapy is based on simultaneous irradiation and MRI data acquisition, and MRI forms an image from the signals induced in RF coils, RIC induced in the MRI RF coils could be detrimental to the MRI signal to noise ratio and introduce image artifacts. However, accurate images are necessary for the success of real-time image guided radiotherapy.

It is therefore an object of the invention to at least mitigate the disadvantages encountered when the treatment beam of a linear accelerator is incident on at least part of a radiofrequency detector coil of an MRI apparatus.

### Summary of the Invention

The invention is as defined in the appended claims.

In accordance with an aspect, there is provided a radiation therapy system comprising:
a radiation source capable of generating a beam of radiation;
a magnetic resonance imaging (MRI) apparatus comprising at least one radiofrequency detector coil; and
an electrically grounded dielectric material between the radiation source and the radiofrequency detector coil for shielding the at least one radiofrequency detector coil from the beam of radiation.

Shielding the at least one radiofrequency detector coil from the beam of radiation with an electrically grounded dielectric material significantly reduces the radiation induced current in the at least one radiofrequency detector coil, and therefore significantly reduces the amount of interference in the MRI images due to radiation.

In an embodiment, the dielectric material has substantially the same density as that of the detector coil.

In an alternative embodiment, the dielectric material has a density that is substantially different from that of the detector coil.

According to another aspect, there is provided a radiofrequency detector coil for a magnetic resonance imaging (MRI) apparatus sheathed at least in part by a dielectric material that is adapted to be electrically grounded.

In one embodiment, only a part of the radiofrequency detector coil upon which a radiation beam would be incident is sheathed by the dielectric material.

### Brief Description of the Drawings

Figures 1 and 2 are graphs showing levels of radiation induced current in two different MRI RF coils in different current scales;
Figures 3 and 4 are perspective schematic views of image guided radiation therapy systems;
Figure 5 is a schematic view of a radiation therapy beam incident on an MRI RF coil;
Figure 6 is a schematic view of a radiofrequency detector coil with one of its windings having been sheathed in an electrically grounded dielectric material;
Figure 7 shows a simulation setup for simulating results of various buildup materials in conjunction with various detector materials;
Figure 7a is a graph showing results from the simulation setup of Figure 7;
Figure 8 is a schematic diagram of a measurement setup constructed to mimic the simulations depicted in Figures 7 and 7a;
Figure 8a is a graph of measurement results obtained from the measurement setup of Figure 8;
Figure 9 is a graph showing the reduction in radiation induced current in different thicknesses of detector material with polytetrafluoroethylene (PTFE) buildup for shielding;
Figure 10 is a graph showing measured and simulated reductions in radiation induced current for a setup with a copper detector and a copper buildup;
Figure 11 is a graph showing measured and simulated reductions in radiation induced current for a setup with a copper detector and an aluminized PTFE buildup;
Figure 12 is a graph showing measuring and simulated reductions in radiation induced current for a setup with a copper detector and PTFE buildup above about 0.16 centimetres;
Figure 13 is a schematic diagram of a measurement setup constructed to observe radiation induced current in an RF coil with various buildups;
Figure 14 is a graph showing increased reduction in radiation induced current in an aluminum coil as the thickness of grounded PTFE buildup is increased;
Figure 15 is a graph showing results of an experiment for RIC reduction when low density material is between a high density coil conductor such a copper and the patient; and
Figure 16 is a graph showing results of an experiment for RIC reduction when the coil conductor is of substantially lower density than copper.

### Detailed Description of the Embodiments

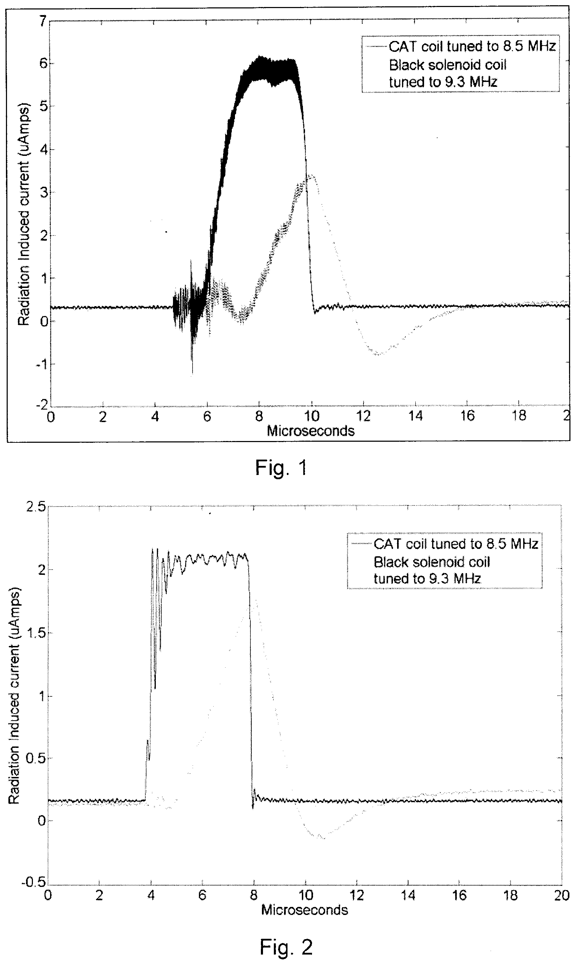

An investigation of radiation induced current in MRI RF coils was reported in "Radiation Induced Currents in MRI RF Coils: Application to Linac/MRI Integration" (B Burke, BG Fallone, S Rathee; 2010 Institute of Physics and Engineering in Medicine; Phys Med. Biol. 55 (2010) 735-746, which is incorporated entirely herein by reference. This work showed that RIC, or Compton current, is present in MRI RF coils when exposed to the pulsed radiation of a linear accelerator beam. Figures 1 and 2 are reproduced from that work, and show the Compton current induced in two MRI RF coils on a Varian 600C linear accelerator, and a Varian Clinac 23iX linear accelerator, respectively.

It has been found that shielding the radiofrequency detector coils of the MRI imaging system with a grounded dielectric material can significantly reduce or eliminate the net loss of electrons from the coil material when the treatment beam is incident directly on the detector coils. This shielding in turn significantly reduces or eliminates the radiation induced current in the detector coils, and accordingly reduces or eliminates the interference in MRI image quality caused by this phenomenon. While in some embodiments shielding is provided by sheathing part or all of the radiofrequency detector coil with the grounded dielectric material, it will be understood that shielding may be done in other manners suitable for compensating for, or preventing, loss of electrons in the coil material upon impact of radiation thereby to reduce or eliminate net loss of electrons due to radiation and as a result significantly reduce or eliminate the amount of current induced in the coil by radiation.

U.S. Patent No. 7,394,254 to Reike et al. entitled "Magnetic Resonance Imaging Having Radiation Compatible Radiofrequency Coils" describes an x-ray system that uses a coil material with a density lower than that of the copper material that is typically used. This is done because the copper coils appear in the radiographic images due to their high density, and the lower energy (kilowatt level) x-rays used for radiographic imaging are significantly attenuated by the copper coil windings. However, such lower-density coils are unsuitable for MRI imaging. Also, the patent is focused on the problem of x-ray signal attenuation and does not contemplate the phenomenon of radiation induced current nor provide any solution suitable for dealing with it.

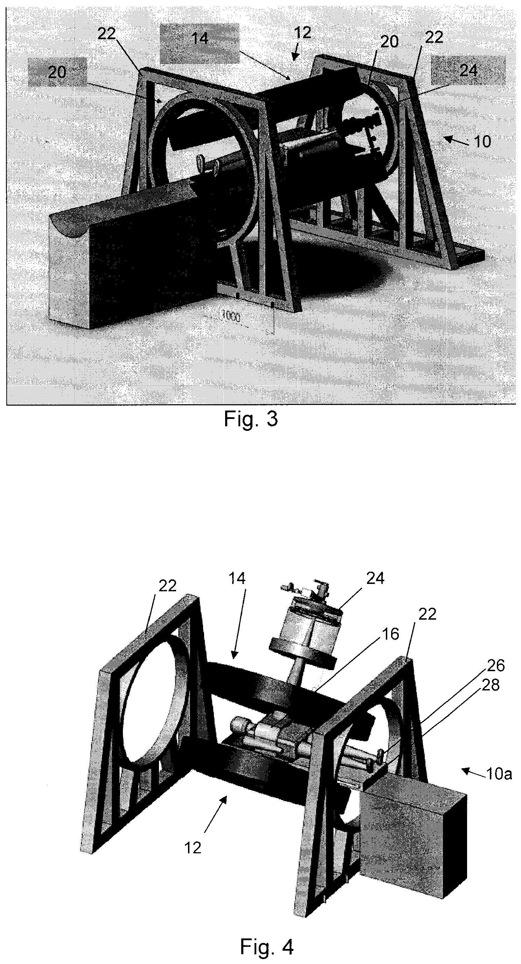

Figures 3 and 4 are perspective schematic views of radiation therapy system 10 according to embodiments. In Figure 4, the radiation therapy system 10 includes an MRI apparatus 12 having a split solenoid magnet 14 and a radiofrequency detector coil 16 positioned about a patient 26 on a couch 28. The split solenoid magnet 14 is mounted on rotational gantries 20 each rotationally supported on a respective frame 22. A radiation source, in this embodiment a linear accelerator 24, is positioned to direct a beam of radiation in a direction parallel to magnetic field lines of the split solenoid magnet 14 for treatment of the patient 26. Figure 3 shows an embodiment in which the beam of radiation is directed perpendicular to the magnetic field lines of the split solenoid magnet..

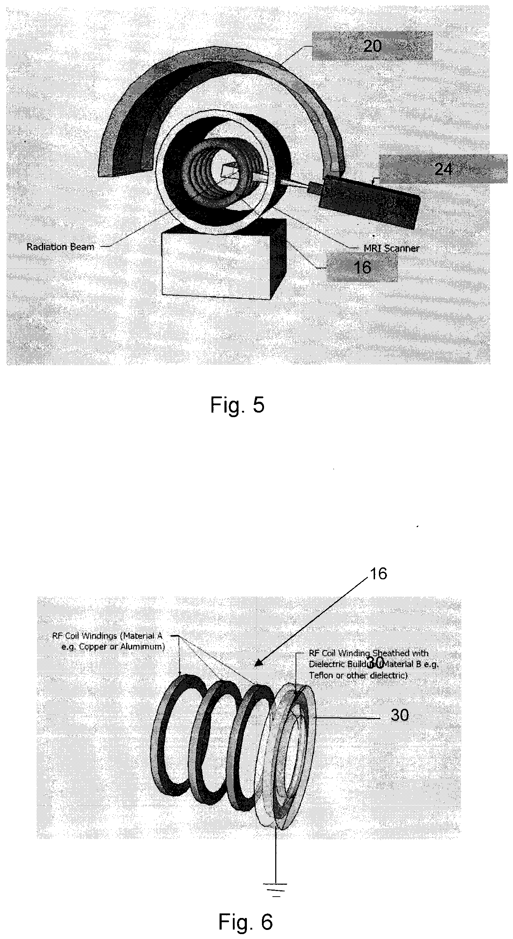

As shown in Figures 4 and 5, the radiation treatment beam generated by the linear accelerator 24 can be incident on radiofrequency detector coil 16 of the MRI apparatus 12 during treatment and imaging. Without shielding, due to the radiation treatment beam being incident on the radiofrequency detector coil 16 of the MRI apparatus 12 during imaging, an interfering Compton current is induced in the radiofrequency detector coil 16, resulting in a compromise of the image quality.

Figure 6 shows a schematic view of a radiofrequency detector coil 16, with one of its windings having been sheathed in, or shielded by, an electrically grounded dielectric material 30. In this embodiment, the coil 16 is formed of copper, and the dielectric material is a buildup of polytetrafluoroethylene (PTFE), a material known more commonly by its trade name Teflon. Provision of such shielding on all parts of the detector coil 16 that can have the radiation treatment beam incident thereon accordingly provides a reduction in the radiation induced current. It will be understood that the radiation beam may not be incident on the entire detector coil 16 and, as such, sheathing may only be required for those portions of the entire detector coil 16 upon which radiation would be incident. However, sheathing on the entire detector coil 16 may be provided.

It is has been found that the most significant reductions in the occurrence and degree of radiation induced current are achieved when the dielectric material is of a similar density to the coil material. However, it has been found that substantial decreases in the amount of radiation induced current result from shielding with dielectric materials having densities that are substantially different from that of the coil material. Furthermore, a small Compton current may not adversely affect imaging to a very high degree, because the signal-to-noise ratio remains sufficiently high.

It has also been observed through simulation that if Copper coil material is not too thin, the use of the dielectric shielding material can substantially eliminate the Compton current in the coil.

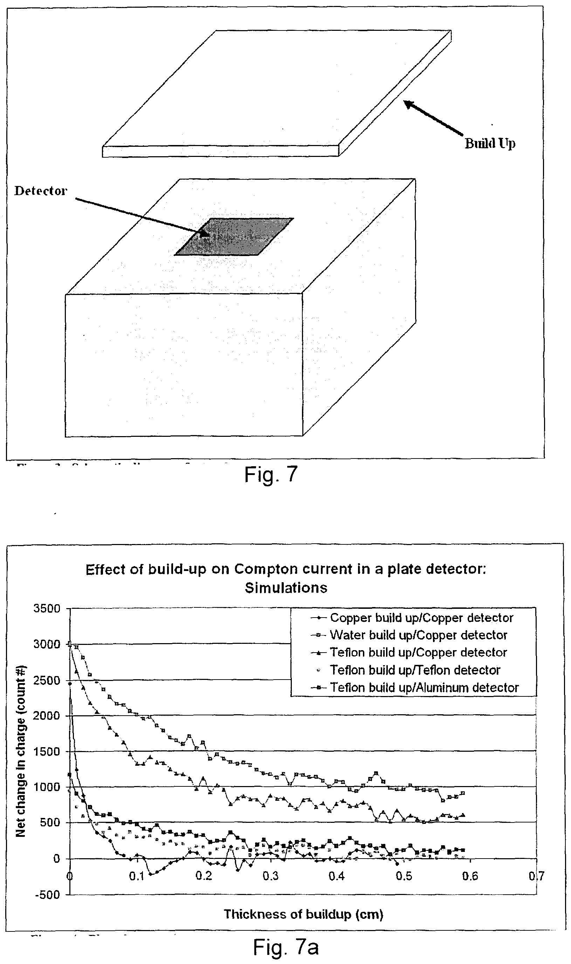

The above observations were based on a setup for computer simulation. The basic simulation setup is as shown in Figure 7. A thin plate of material A (a conductor suitable for RF coils) as a detector would be placed on a slab of buildup material B and exposed to a pulsed radiation beam. The Compton current induced in material A would be measured, and measurements repeated as increasing thicknesses of material B were to be piled on top of detector material A as buildup material. The simulation setup allowed the variation of both the detector material A and the buildup material B, and permitted examination of three scenarios: 1) materials A and B are the same; 2) materials A and B are different and have significantly different densities, and 3) materials A and B are different but have similar densities.

A previously benchmarked computer simulation program for radiation interactions with materials called PENELOPE (Sempau et al 1997) was used to calculate the Compton current in detector material A for the three scenarios. During the simulations, a 6 MeV photon beam, as is commonly used in radiation therapy, was directed from the top onto the detector material A, as shown in Figure 7. A proxy for the resulting Compton current was ascertained based on the net loss of electrons from the detector material (count number). The results of the simulations, shown in the Figure 7a graph, indicate that in scenario 1 the Compton current goes to zero as the thickness of the buildup material is increased, as seen in the Copper build up/Copper detector and the Teflon build up/Teflon detector cases. In scenario 2, the induced current decreases initially but does not reach a zero value even at larger buildup thicknesses, as seen in the water build up/copper detector and Teflon build up/Copper detector cases. In scenario 3, where the buildup material and detector material have similar densities, the Compton current drops to a near zero level, as seen in the Teflon build up/aluminum detector case. It was predicted that the near-zero value seen in scenario 3 simulation would be zero also in a practical measurement, so a setup was constructed to mimic the simulations.

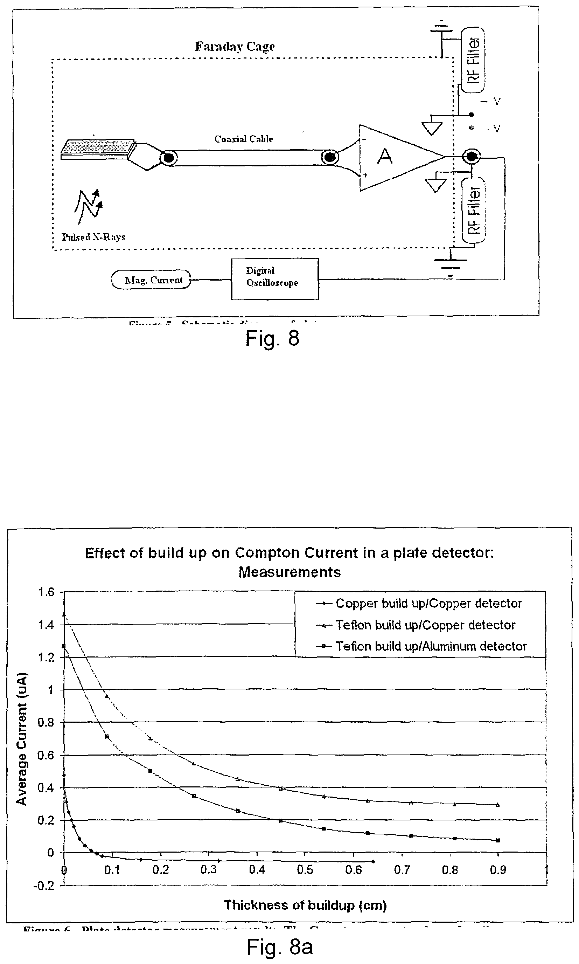

The measurement setup constructed to mimic the simulations is shown in schematic form in Figure 8. The measurement system was placed inside of a Faraday type RF cage to shield the measurements from unwanted RF noise produced by medical linacs (Burke et al. 2009). This RF noise would otherwise dominate the measurement signal, which would result in a situation in which accurate measurement of Compton current would not be possible. The detector plate was connected to an amplifier via a coaxial cable, and the build up material was grounded and electrically isolated from the detector. The RF cage was placed on the treatment couch of the linac, and exposed to pulsed radiation to induce Compton current in the RF coil. The amplifier was not irradiated. The Compton current was measured with an oscilloscope. The results of the measurements are shown in the graph of Figure 8a, and are similar to the results of the simulations shown in Figure 7a. That is, when the detector and buildup material are the same, the Compton current goes to zero, as seen in the Copper build up/Copper detector measurement. When the two materials have significantly different densities, the Compton current converges to a non-zero value, as seen in the Teflon build up/Copper detector measurement. When the two materials are different but have similar densities, the Compton current again goes to a value which is nearly zero, as seen in the Teflon build up/aluminum detector measurement.

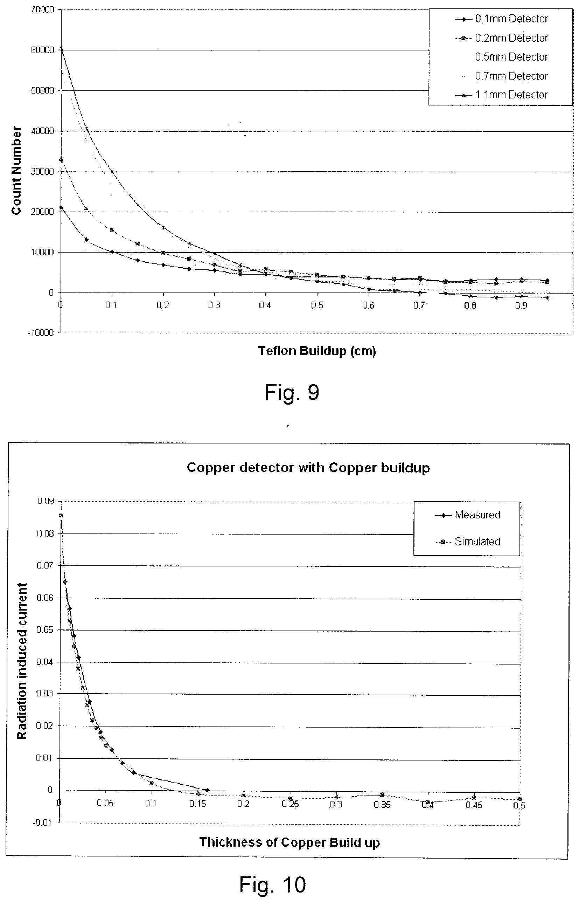

Figure 9 is a graph showing that, in simulations, Compton current in thin copper having 0.1 and 0.2 millimetre thicknesses is not fully eliminated despite the thickness of a Teflon buildup. However, Compton current in copper having 0.5 millimetre and higher thicknesses can, in simulations, be substantially eliminated with sufficient buildup thickness.

Figures 10 through 12 are graphs showing the results of further experiments to reproduce the results of the simulations plotted in Figure 9. In particular, Figure 10 is a graph showing reduction to zero of radiation induced current in copper plate material for thicknesses of copper buildup material above about 0.16 centimetres (measured). It will be noted that, where coils are concerned in an imaging system, a metal or otherwise conductive buildup material cannot be used since it will interfere significantly with imaging. In particular, placing metal build up near an MR coil can alter the Q factor and/or the resonant frequency of the coil, and as a consequence can lower the signal-to-noise ratio of the acquired images substantially (up to 20% as disclosed in Ha S et al. 2010 Development of a new RF coil and γ-ray radiation shielding assembly for improved MR image quality in SPECT/MRI. Phys. Med. Biol. 55 2495-2504), thus yielding lower quality images. For this reason, a dielectric material is preferable for shielding the radiofrequency coil from incident radiation, over metal or otherwise conductive material.

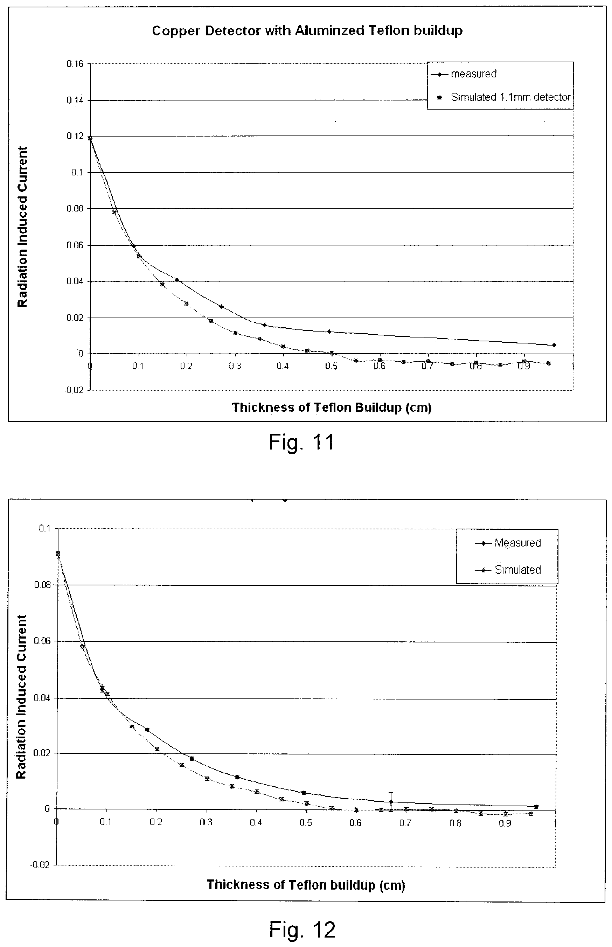

Figure 11 is a graph showing reduction to zero of radiation induced current in copper plate material for thicknesses of aluminized Teflon buildup above about 0.16 centimetres (simulated). The measured radiation induced current shown in Figure 11 does not go all the way down to zero, but is reduced enough to produce relatively insignificant levels of noise due to RIC. Figure 12 is a graph showing reduction to zero of radiation induced current in aluminum plate material for thicknesses of Teflon buildup above about 0.55 centimetres (simulated). The measured radiation induced current shown in Figure 12 does not go to zero, but is reduced enough to produce relatively insignificant levels of noise.

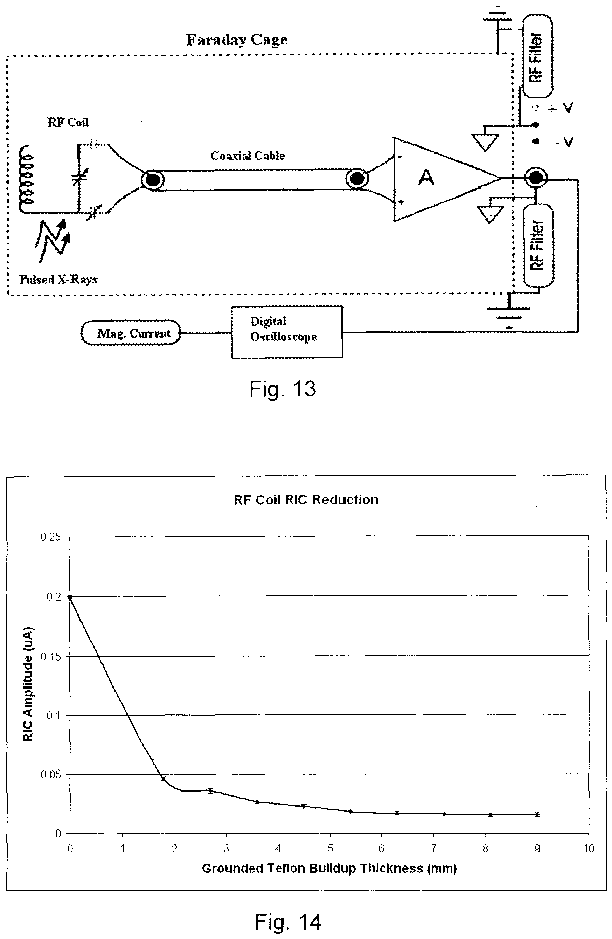

The measurement setup constructed to observe radiation induced current in an RF coil with various buildups, as opposed to a plate, is shown in Figure 13.

Figure 14 is a graph showing an increase in reduction of radiation induced current in an aluminum coil as the thickness of the grounded Teflon buildup is increased. The reduction levels off at a RIC current amount that is about 90% less than without the buildup.

In an alternative embodiment, the coil 16 could be formed of another conductive material of sufficient density to facilitate MRI imaging.

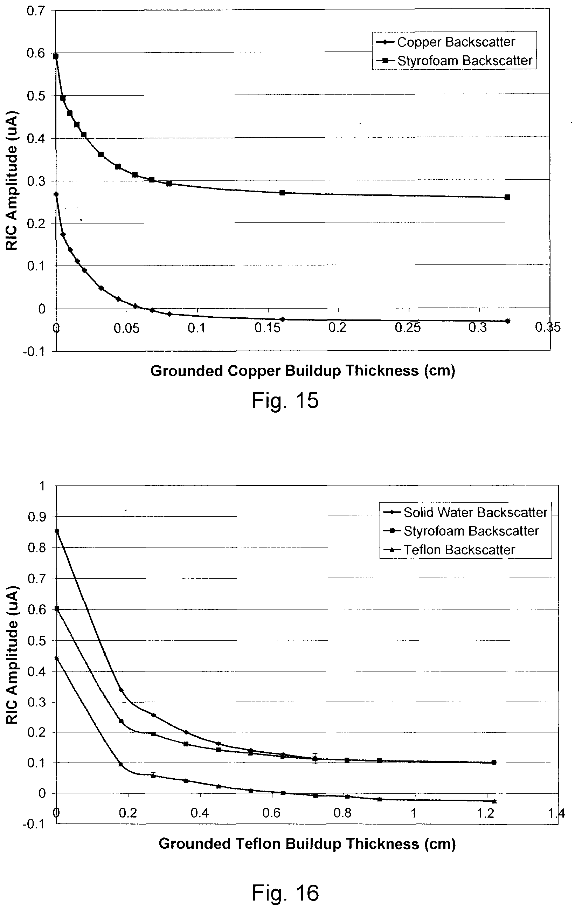

Figure 15 is a graph showing results of an experiment for RIC current reduction when low density material is between the high density coil conductor and the patient. The data in Figure 15 shows that if there exists low density material such as air (simulated by Styrofoam in the experiment) between a coil conductor having substantially high density (such as copper as used in the experiment) and the patient, then the RIC current is not reduced to significantly low levels by grounded buildup material. This is the case even when the buildup material is the same as used in the coil conductor. "Backscatter" in the graph of Figure 15 signifies the material that occupies the space in the gap (if there is any) between the coil conductor and the patient.

Figure 16 is a graph showing RIF current reduction when the coil conductor is of lower density. The data depicted in Figure 16 shows that for coil conductors of lower density such as aluminum, the reduction in RIC current by the grounded dielectric buildup material, such as Teflon, is always significant irrespective of the type of material occupying the space in the gap between the coil conductor and the patient. Teflon backscatter shows the result in the event that the patient was substantially the same as Teflon in density. As will be appreciated, a patient would not be substantially the same density as Teflon, so this result while informative is unrealistic. Solid water backscatter shows the result in the event that the patient has a similar density to solid water (which is realistic), with the coil conductor being in contact with the solid water. That is, there is no gap. Styrofoam, which is used to simulate the density of air, refers to there being a gap between the conductor coil and the patient.

A radiofrequency detector coil 16 with suitable shielding as described herein could be formed as a separate unit for installation in an image guided radiotherapy (IGRT) system. Alternatively, material for shielding could be provided as a separate option for coupling with a coil at the time of installation of an IGRT system.

Although embodiments have been described, those of skill in the art will appreciate that variations and modifications may be made without departing from the purpose and scope thereof as defined by the appended claims.

### References

Abdel-Rahman W, Seuntjens J P, Verhaegen F and Podgorsak E B 2006 Radiation induced currents in parallel plate ionization chambers: measurement and Monte Carlo simulation for megavoltage photon and electron beams Med. Phys. 33 3094-104
Burke B, Lamey M, Rathee S, Murray B and Fallone B G 2009 Radio frequency noise from clinical linear accelerators Phys. Med. Biol. 54 2483-92
Dawson L A and Jaffray D A 2007 Advances in image-guided radiation therapy J. Clin. Oncol. 25 938-46
Degenhart H J and Schlosser W 1961 Transient effects of pulsed nuclear radiation on electronic parts and materials IRE Trans. Compon. Parts 8 123-8
Fallone B G, Carlone M, Murray B, Rathee S, Stanescu T, Steciw S, Wachowicz K and Kirkby C 2007 Development of a linac-MRI system for real-time ART Med. Phys. 34 2547
Fallone B G, Murray B, Rathee S, Stanescu T, Steciw S, Vidakovic S, Blosser E and Tymofichuk D 2009 First MR images obtained during megavoltage photon irradiation from a prototype integrated linac-MR system Med.Phys. 36 2084-8
Johns H E, Aspin N and Baker R G 1958 Currents induced in the dielectrics of ionization chambers through the action of high-energy radiation J. Radiat. Res. 9 573-88
Karzmark C J, Nunan C S and Tanabe E 1993 Medical Electron Accelerators (New York: McGraw-Hill)
Kirkby C, Stanescu T, Rathee S, Carlone M, Murray B and Fallone B G 2008 Patient dosimetry for hybrid MRlradiotherapy systems Med. Phys. 35 1019-27
Lagendijk J J, Raaymakers B W, Van Der Heide U A, Overweg J, Brown K J, Bakker C, Raaijmakers A J, Vulpen M, Welleweerd J and Jurgenliemk-Schulz I 2005 In room magnetic resonance imaging guided ratiotherapy (MRIgRT) Med. Phys. 32 2067
Meyer R A, Bouquet F L and Alger R S 1956 Radiation induced conductivity in polyethylene and teflon J. Appl.Phys. 27 1012-8
Raaijmakers A J, Raaymakers B W and Lagendijk J J 2008 Magnetic field-induced dose effects in MR-guided radiotherapy systems: dependence on the magnetic field strength Phys. Med. Biol. 53 909-23
Raaymakers B W et al 2009 Integrating a 1.5 T MRI scanner with a 6 MV accelerator: proof of concept Phys. Med. Biol. 54 N229-37
Sato F, Tanaka T, Kagawa T and Iida T 2004 Impedance measurements of thin film ceramics under ion beam irradiation J. Nucl. Mater. 329-333 1034-7
Ha S et al. 2010 Development of a new RF coil and γ-ray radiation shielding assembly for improved MR image quality in SPECT/MRI. Phys. Med. Biol. 55 2495-2504

## Claims

1. A radiofrequency detector coil (16) for a magnetic resonance imaging (MRI) apparatus, at least a portion of the radiofrequency detector coil (16) sheathed by an adjoining electrically grounded dielectric material (30), wherein the dielectric material (30) has a density that is substantially similar to that of the radiofrequency detector coil (16).

2. The radiofrequency detector coil (16) of claim 1, wherein the radiofrequency detector coil (16) is formed of aluminum and the dielectric material (30) is formed of polytetrafluoroethylene.

3. The radiofrequency detector coil (16) of claim 1, wherein only a part of the the radiofrequency detector coil (16) is sheathed by the dielectric material (30).

4. The radiofrequency detector coil (16) of claim 1, wherein the electrically grounded dielectric material (30) has a thickness selected to provide a minimal loss of electrons.

5. The radiofrequency detector coil (16) of claim 4, wherein the thickness is selected to provide electronic equipibrium.

6. The radiofrequency detector coil (16) of claim 1, wherein the electrically grounded dielectric material (30) is grounded using a point connection.

7. The radiofrequency detector coil (16) of claim 3, wherein at least one winding of the radiofrquency detector coil (16) is sheathed by the grounded dielectric material (30).

8. A radiation therapy system (10) comprising:
a radiation source (24) capable of generating a beam of radiation; and
a magnetic resonance imaging (MRI) apparatus (12) comprising at least one radiofrequency detector coil (16) as defined in any one of claims 1 to 7.

## Patentansprüche

1. Radiofrequenz-Detektorspule (16) für eine Magnetresonanzbildgebungsvorrichtung (MRI), wobei mindestens ein Teil der Radiofrequenz-Detektorspule (16) von einem angrenzenden, elektrisch geerdeten dielektrischen Material (30) umhüllt ist, wobei das dielektrische Material (30) eine Dichte aufweist, die der der Radiofrequenz-Detektorspule (16) im Wesentlichen ähnlich ist.

2. Radiofrequenz-Detektorspule (16) nach Anspruch 1, wobei die Radiofrequenz-Detektorspule (16) aus Aluminium gebildet ist und das dielektrische Material (30) aus Polytetrafluorethylen gebildet ist.

3. Radiofrequenz-Detektorspule (16) nach Anspruch 1, wobei nur ein Teil der Radiofrequenz-Detektorspule (16) von dem dielektrischen Material (30) umhüllt ist.

4. Radiofrequenz-Detektorspule (16) nach Anspruch 1, wobei das elektrisch geerdete dielektrische Material (30) eine Dicke aufweist, die so ausgewählt ist, dass ein minimaler Verlust von Elektronen gewährleistet wird.

5. Radiofrequenz-Detektorspule (16) nach Anspruch 4, wobei die Dicke so ausgewählt ist, dass ein elektronisches Gleichgewicht gewährleistet wird.

6. Radiofrequenz-Detektorspule (16) nach Anspruch 1, wobei das elektrisch geerdete dielektrische Material (30) unter Verwendung einer Punktverbindung geerdet wird.

7. Radiofrequenz-Detektorspule (16) nach Anspruch 3, wobei mindestens eine Wicklung der Radiofrequenz-Detektorspule (16) von dem geerdeten dielektrischen Material (30) umhüllt ist.

8. Strahlentherapiesystem (10), umfassend:
eine Strahlungsquelle (24), die einen Strahlungsstrahl erzeugen kann; und
eine Magnetresonanzbildgebungsvorrichtung (MRI) (12), die mindestens eine Radiofrequenz-Detektorspule (16) nach einem der Ansprüche 1 bis 7 aufweist.

## Revendications

1. Bobine de détection de radiofréquence (16) destinée à un appareil d'imagerie par résonance magnétique (IRM), au moins une partie de la bobine de détection de radiofréquence (16) étant gainée par un matériau diélectrique à la masse électriquement adjacent (30), dans laquelle le matériau diélectrique (30) a une densité qui est substantiellement similaire à celle de la bobine de détection de radiofréquence (16).

2. Bobine de détection de radiofréquence (16) selon la revendication 1, dans laquelle la bobine de détection de radiofréquence (16) est formée d'aluminium et le matériau diélectrique (30) est formé de polytétrafluoroéthylène.

3. Bobine de détection de radiofréquence (16) selon la revendication 1, dans laquelle une partie seulement de la bobine de détection de radiofréquence (16) est gainée par le matériau diélectrique (30).

4. Bobine de détection de radiofréquence (16) selon la revendication 1, dans laquelle le matériau diélectrique électriquement à la masse (30) a une épaisseur sélectionnée pour fournir une perte minimale en électrons.

5. Bobine de détection de radiofréquence (16) selon la revendication 4, dans laquelle l'épaisseur est sélectionnée pour fournir un équilibre électronique.

6. Bobine de détection de radiofréquence (16) selon la revendication 1, dans laquelle le matériau diélectrique électriquement à la masse (30) est relié à la terre au moyen d'un point de connexion.

7. Bobine de détection de radiofréquence (16) selon la revendication 3, dans laquelle au moins un enroulement de la bobine de détection de radiofréquence (16) est gainé par le matériau diélectrique à la masse (30).

8. Système de radiothérapie (10) comprenant :
une source de radiation (24) capable de générer un faisceau de radiation ; et
un appareil d'imagerie par résonance magnétique (IRM) comprenant au moins une bobine de détection de radiofréquence (16) telle que définie dans l'une quelconque des revendications 1 à 7.
